# EUROPÄISCHE PATENTSCHRIFT

(11) **EP 0 727 189 B1**
(45) Veröffentlichungstag und Bekanntmachung des Hinweises auf die Patenterteilung: **01.04.1998**
(21) Anmeldenummer: 96100328.2
(22) Anmeldetag: 11.01.1996
(51) Int. Cl.: A61B 17/74

(54) **Vorrichtung zur Versorgung von Knochenbrüchen**
Device for treating bone fractures
Dispositif pour traiter des fractures d'os

(30) Priorität: 18.02.1995 DE 19505609
(43) Veröffentlichungstag der Anmeldung: 21.08.1996
(73) Patentinhaber: AESCULAP AG, 78532 Tuttlingen (DE)
(72) Erfinder: Stedtfeld, Hans-Werner, D-90475 Nürnberg (DE); Lacher, Rainer, D-78570 Mülheim (DE); Saueressig, Thomas, D-78532 Tuttlingen (DE)
(74) Vertreter: Hoeger, Stellrecht & Partner

(56) Entgegenhaltungen:
- EP-A- 0 441 577
- EP-A- 0 586 824
- DE-U- 9 206 580
- FR-A- 2 342 710
- US-A- 2 699 774
- US-A- 3 530 854
- US-A- 4 095 591
- US-A- 4 657 001

## Beschreibung

Die Erfindung betrifft eine Vorrichtung zur Versorgung von Knochenbrüchen im gelenknahen proximalen Teil des Femurknochens mit einem in den Markraum des Femurknochens einsetzbaren Verriegelungsnagel, der in seinem oberen Bereich eine schräg zu seiner Längsachse verlaufende Durchgangsöffnung aufweist, mit einer in dieser Durchgangsöffnung in axialer Richtung und gegen Drehung um die Längsachse festgelegten Hülse und mit einer mit einem Schaft in diese Hülse eintauchenden und in der Hülse axial verschieblich aufgenommenen Schenkelhalsschraube.

Eine solche Vorrichtung ist beispielsweise aus der EP 0 441 577 A2 bekannt.

Bei dieser Vorrichtung und bei anderen vergleichbaren bekannten Vorrichtungen ergibt sich ein Kontakt zwischen dem Femurkopf einerseits und der Vorrichtung zur Versorgung des Knochenbruches andererseits ausschließlich über die Schenkelhalsschraube, die in den Femurkopf eingeschraubt ist. Obwohl die Schenkelhalsschraube bei bekannten Vorrichtungen dieser Art in dem Verriegelungsnagel drehfest gehalten ist, kann eine Verdrehung des Femurkopfes gegenüber dem Femur nicht mit Sicherheit ausgeschlossen werden, da sich eine solche Verdrehung auch dadurch ergeben kann, daß sich die Einschraubtiefe der Schenkelhalsschraube in den Femurkopf geringfügig verändert, daß sich also der Femurkopf auf der Schenkelhalsschraube mehr oder weniger verdreht.

Eine ähnliche gattungsgemäße Vorrichtung ist auch aus der DE-U-92 06 580 bekannt. Obwohl dort die die Schenkelhalsschraube umgebende Hülse verlängert werden kann, wird der Femurkopf doch ausschließlich von der Schenkelhalsschraube selbst gestützt, so daß auch in diesem Falle eine Verdrehung des Schenkelhalskopfs gegenüber der Schenkelhalsschraube nicht ausgeschlossen werden kann.

Es ist Aufgabe der Erfindung, eine gattungsgemäße Vorrichtung so auszugestalten, daß diese Gefahr einer Verdrehung des Femurkopfs gegenüber dem Femur mit Sicherheit ausgeschlossen wird, wobei zusätzlich eine Annäherung des Femurkopfs an den Femurknochen in beschränktem Umfange ermöglicht werden soll.

Diese Aufgabe wird bei einer Vorrichtung der eingangs beschriebenen Art erfindungsgemäß dadurch gelöst, daß die Hülse auf der der Schenkelhalsschraube zugewandten Seite so weit vorsteht, daß sie in den Femurkopf hineinragt, daß sie in diesem Teil auf ihrem Außenumfang Vorsprünge und Rücksprünge trägt, die in Umfangsrichtung unterschiedliche Durchmesser der Hülse erzeugen, und daß die Eintauchtiefe der Schenkelhalsschraube in die Hülse durch einen Anschlag der Schenkelhalsschraube begrenzt ist, dessen Abstand von der Hülse beim Anlegen der Vorrichtung zwischen 2 und 12 mm positionierbar ist.

Die Hülse wird also als Verdrehsicherung benutzt. Sie taucht mit ihrer profilierten Umfangsfläche in den Femurkopf ein und gräbt sich dabei in die relativ weiche Knochenmasse im Innern des Femurkopfes, wobei durch die Umfangsprofilierung eine Verdrehung des Femurkopfes gegenüber der Hülse mit Sicherheit verhindert wird. Durch den Anschlag der Schenkelhalsschraube ist sichergestellt, daß die Annäherung des Femurkopfs an den Femur ein bestimmtes Maß nicht überschreitet, denn bei der Anlage des Anschlags an der Hülse übernimmt auch die Schenkelhalsschraube durch ihre Einschraubung in den Femurkopf eine zusätzliche Tragfunktion und sichert den Femurkopf gegen eine weitere Annäherung.

Eine besonders sichere Drehfixierung läßt sich erreichen, wenn gemäß einer bevorzugten Ausführungsform der Erfindung die Vorsprünge an ihren achsfernsten Stellen achsparallele scharfe Kanten aufweisen. Diese graben sich in die Knochenmasse ein und sichern den Femurkopf gegen eine Rotation.

Insbesondere kann vorgesehen sein, daß die Vor- und Rücksprünge durch achsparallele Nuten gebildet sind, die längs des Umfanges der Hülse angeordnet sind. Bei einer ersten Ausführungsform weisen die Nuten eine über ihre Länge gleichbleibende Tiefe auf. Dies führt dazu, daß die Kräfte, die die in den Femurkopf eindringende Hülse in axialer Richtung auf den Femurkopf ausübt, im wesentlichen konstant bleiben, wenn der Femurkopf beim Heilungsprozeß sich in axialer Richtung geringfügig an den Femur annähert. Diesen Vorgang bezeichnet man als "aufsintern", er kann dazu führen, daß bei zu starkem Aufsintern nach der Heilung eine Fehlstellung des Femurkopfes auftritt.

Bei einer anderen bevorzugten Ausführungsform ist vorgesehen, daß die Tiefe der Nuten vom freien Ende der Hülse ausgehend abnimmt. Dadurch nehmen die Kräfte zu, die die Hülse beim weiteren Eindringen in den Femurkopf in axialer Richtung aufnimmt, d. h. der Widerstand gegen das weitere Annähern des Femurkopfes an den Femur nimmt mit zunehmender Annäherung zu. Dadurch wird das Aufsintern im Verlauf des Heilungsprozesses immer stärker behindert, so daß Fehlstellungen vermieden werden können.

Günstig ist es, wenn die Nuten eine kreisbogenförmigen Querschnitt aufweisen.

Es kann auch vorgesehen sein, daß die Breite der Nuten vom freien Ende der Hülse ausgehend abnimmt.

Dabei ist es vorteilhaft, wenn der Anschlag von dem Gewinde der Schenkelhalsschraube gebildet wird.

Der Operateur kann durch die Länge der Hülse oder durch die Lage der Hülse im Verriegelungsnagel den Abstand des Anschlages von der Hülse bestimmen, dieser liegt erfindungsgemäß zwischen 2 und 12 mm.

Die Drehsicherung der profilierten Hülse ist in der Regel so gut, daß die Schenkelhalsschraube in der Hülse frei drehbar gelagert sein kann.

Trotzdem kann eine noch weitergehende Drehsicherung dadurch erreicht werden, daß die Schenkelhalsschraube in der Hülse gegen eine Drehung um die Längsachse gesichert ist. Dies könnte dann von Bedeutung sein, wenn der Femurkopf im Bereich des Eintauchens der Hülse beschädigt ist oder nur wenig zur Drehsicherung beitragendes Knochenmaterial aufweist.

Die nachfolgende Beschreibung einer bevorzugten Ausführungsform der Erfindung dient im Zusammenhang mit der Zeichnung der näheren Erläuterung. Es zeigen:

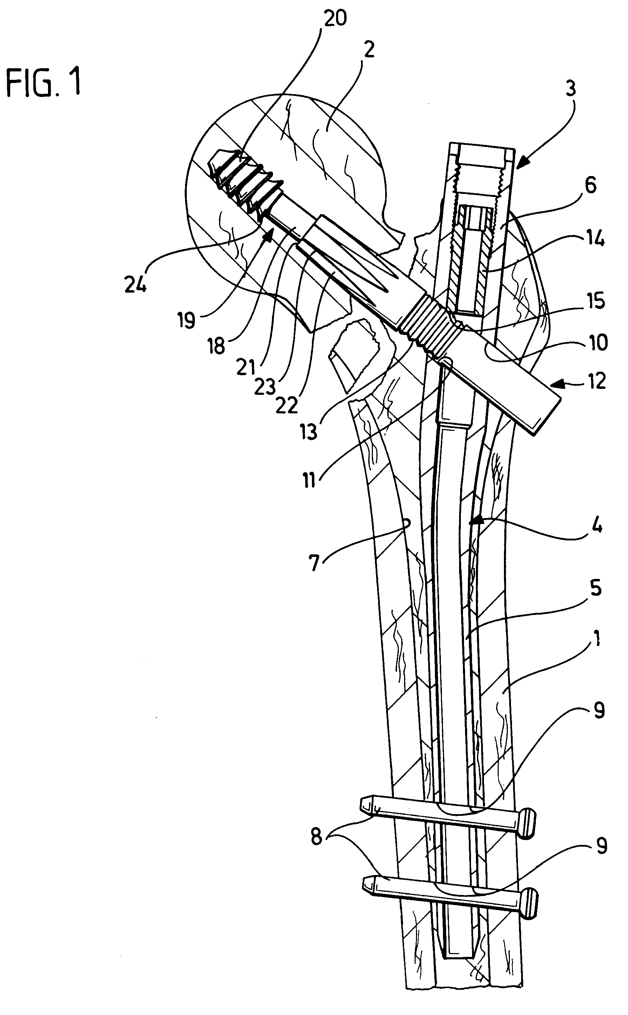

- Figur 1:: eine Längsschnittansicht durch den proximalen Teil eines Femurs mit einer eingesetzten Vorrichtung zur Versorgung eines gelenknahen Knochenbruches,
- Figur 2:: eine Längsschnittansicht der Hülse der Vorrichtung der Figur 1 und
- Figur 3:: eine Ansicht dieser Hülse von der Vorderseite her.

In der Zeichnung ist der obere, proximale Teil eines Femurknochens 1 dargestellt, dessen Femurkopf 2 durch eine Fraktur von ihm getrennt ist.

Für den Heilungsprozeß muß dieser Femurkopf 2 relativ zum Femurknochen 1 dauerhaft fixiert werden, und dazu ist in den Femurknochen eine Vorrichtung 3 zur Versorgung des Knochenbruches eingesetzt. Diese umfaßt zunächst einen Verriegelungsnagel 4 mit einem rohrförmigen, an der Unterseite offenen Schaft 5 und einem ebenfalls rohrförmigen Oberteil 6. Die Längsachsen des Schaftes 5 und des Oberteiles 6 sind geringfügig gegeneinander geneigt, beispielsweise um einen Winkel von 15°. Dies Wandstärke des Schaftes 5 ist wesentlich geringer als die Wandstärke des Oberteiles 6, so daß der Schaft 5 elastischer ausgebildet ist als das Oberteil 6.

Der Verriegelungsnagel 4 wird von der Oberseite her in den Femurknochen 1 so eingesetzt, daß er mit seinem Schaft 5 in den Markraum 7 des Femurknochens 1 eintaucht. In dieser Lage wird er durch den Femurknochen 1 durchsetzende Verriegelungsschrauben 8 fixiert, die durch miteinander ausgerichtete Öffnungen 9 im Schaft 5 hindurchgesteckt sind.

Im Oberteil 6 des Verriegelungsnagels 4 sind zwei miteinander ausgerichtete Durchbrechungen 10 in der Wand des Verriegelungsnagels 4 vorgesehen, die gemeinsam eine schräg zur Längsachse des Oberteils 6 verlaufende Durchstecköffnung 11 ausbilden. In diese Durchstecköffnung 11 wird durch die Wand des Femurknochens 1 hindurch eine die Durchstecköffnung 11 ausfüllende Hülse 12 eingesteckt, die in Richtung des Schenkelhalses, also der Verbindung zwischen Femurknochen 1 und Femurkopf 2, angeordnet ist. Die Hülse 12 ist so lang ausgebildet, daß sie an ihrem unteren, vom Femurkopf 2 abgewandten Ende aus dem Femurknochen 1 hervorsteht, an ihrem gegenüberliegenden Ende ragt sie bis in das Innere des Femurkopfes 2 vor.

Die Hülse 12 trägt an ihrem Außenumfang in Umfangsrichtung verlaufende Rippen 13, die bei eingesetzter Hülse 12 im Innern des Verriegelungsnagels 4 angeordnet sind. Von der offenen Oberseite her wird in den Schaft 5 eine Fixationsschraube 14 eingeschraubt, die mit ihrem freien Ende 15 an dem mit Rippen 13 versehenen Umfangsbereich der Hülse 12 anliegt und die Hülse dadurch sowohl in axialer Richtung als auch gegen eine Drehung um ihre Längsachse in der Durchstrecköffnung 11 festlegt.

Die Hülse 12 weist eine Durchgangsbohrung 16 auf, die sich am unteren Ende stufenförmig erweitert und in diesem Bereich ein Innengewinde 17 trägt, in das ein in der Zeichnung nicht dargestelltes Einsetzwerkzeug eingeschraubt werden kann.

In die Durchgangsbohrung 16 ist der Schaft 18 einer Schenkelhalsschraube 19 eingeschoben, dieser füllt die Durchgangsbohrung 16 aus und ist in dieser in Längsrichtung frei verschieblich gelagert. Die Schenkelhalsschraube 19 weist ein an den Schaft 18 anschließendes Einschraubgewinde 20 an sich bekannter Bauart auf, welches in das Innere des Femurkopfes 2 eingeschraubt ist.

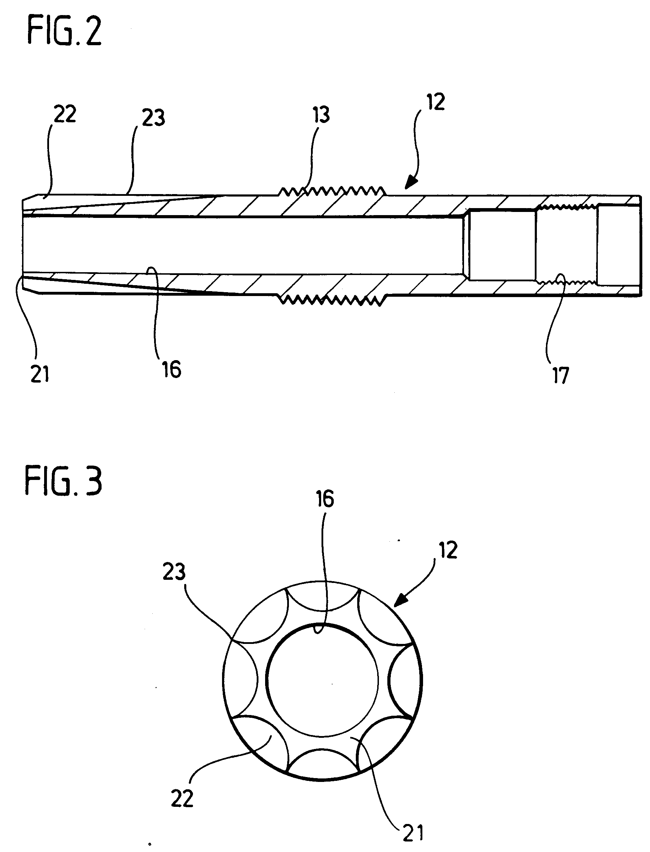

Die Hülse 12 ist in dem Bereich zwischen den Rippen 13 einerseits und ihrem der Schenkelhalsschraube 19 zugewandten Ende 21 andererseits durch achsparallel verlaufende, in Umfangsrichtung nebeneinander angeordnete Nuten 22 profiliert, die bei dem dargestellten Ausführungsbeispiel einen bogenförmigen Querschnitt haben und deren Tiefe vom freien Ende 21 zum Verriegelungsnagel 4 hin abnimmt. Diese Nuten 22 bilden zwischen sich achsparallele scharfe Kanten 23 aus, so daß insbesondere im Bereich des freien Endes 21 ein rosettenartiger Querschnitt entsteht (Figur 3). Dieser profilierte Bereich der Hülse 12 wird in das Innere des Femurkopfes 2 eingedrückt und wirkt dort als Drehsicherung. Dabei graben sich die Kanten 23 in die Knochenmasse des Femurkopfes ein.

Das Einschraubgewinde 20 der Schenkelhalsschraube 19 hat einen Außendurchmesser, der größer ist als der des Schaftes 18. Das der Hülse 12 zugewandte Ende des Einschraubgewindes 20 bildet somit einen Anschlag 24, der bei der Anlage am freien Ende 21 der Hülse 12 das weitere Einschieben der Schenkelhalsschraube 19 in die Hülse 12 begrenzt.

Bei dem Anlegen der Vorrichtung zur Versorgung des Knochenbruches wird dieser Anschlag 24 im Abstand vom freien Ende 21 der Hülse 12 angeordnet, dies läßt sich durch die Verwendung unterschiedlich langer Hülsen 12 oder durch unterschiedliche Positionierung der Hülse 12 in der Durchstecköffnung 11 erreichen. Erfindungsgemäß wird dabei ein Anfangsabstand von 2 bis zu 12 mm verwendet. Dadurch ist es möglich, daß im Verlauf des Heilungsprozesses der Femurkopf 2 sich dem Femurknochen 1 annähert, allerdings wird diese Annäherung dadurch begrenzt, daß der Anschlag 24 am freien Ende 21 der Hülse 12 zur Anlage gelangt.

## Patentansprüche

1. Vorrichtung (3) Versorgung von Knochenbrüchen im gelenknahen proximalen Teil des Femurknochens mit einem in den Markraum des Femurknochens einsetzbaren Verriegelungsnagel (4), der in seinem oberen Bereich eine schräg zu seiner Längsachse verlaufende Durchgangsöffnung (11) aufweist, mit einer in dieser Durchgangsöffnung in axialer Richtung und gegen Drehung um die Längsachse festgelegten Hülse (12) und mit einer mit einem Schaft in die Hülse eintauchenden und in der Hülse axial verschieblich aufgenommenen Schenkelhalsschraube (19), dadurch gekennzeichnet, daß die Hülse (12) auf der der Schenkelhalsschraube (19) zugewandten Seite soweit vorsteht, daß sie in den Femurkopf (2) hineinragt, daß sie in diesem Teil auf ihrem Außenumfang Vorsprünge (23) und Rücksprünge (22) trägt, die in Umfangsrichtung unterschiedliche Durchmesser der Hülse (12) erzeugen, und daß die Eintauchtiefe der Schenkelhalsschraube (19) in die Hülse (12) durch einen Anschlag (24) der Schenkelhalsschraube (19) begrenzt ist, dessen Abstand von der Hülse (12) beim Anlegen der Vorrichtung zwischen zwei und zwölf Millimetern positionierbar ist.

2. Vorrichtung nach Anspruch 1, dadurch gekennzeichnet, daß die Vorsprünge (23) an ihren achsfernsten Stellen achsparallele scharfe Kanten aufweisen.

3. Vorrichtung nach Anspruch 1 oder 2, dadurch gekennzeichnet, daß die Vor- und Rücksprünge durch achsparallele Nuten (22) gebildet sind, die längs des Umfanges der Hülse (12) angeordnet sind.

4. Vorrichtung nach Anspruch 3, dadurch gekennzeichnet, daß die Nuten (22) eine über ihre Länge gleichbleibende Tiefe aufweisen.

5. Vorrichtung nach Anspruch 3, dadurch gekennzeichnet, daß die Tiefe der Nuten (22) vom freien Ende (21) der Hülse (12) ausgehend abnimmt.

6. Vorrichtung nach einem der Ansprüche 3 bis 5, dadurch gekennzeichnet, daß die Nuten (22) einen bogenförmigen Querschnitt aufweisen.

7. Vorrichtung nach einem der Ansprüche 3 bis 6, dadurch gekennzeichnet, daß die Breite der Nuten (22) vom freien Ende (21) der Hülse (12) ausgehend abnimmt.

8. Vorrichtung nach einem der voranstehenden Ansprüche, dadurch gekennzeichnet, daß der Anschlag (24) von dem Gewinde (20) der Schenkelhalsschraube (19) gebildet ist.

9. Vorrichtung nach einem der voranstehenden Ansprüche, dadurch gekennzeichnet, daß die Schenkelhalsschraube (19) in der Hülse (12) frei drehbar ist.

10. Vorrichtung nach einem der Ansprüche 1 bis 8, dadurch gekennzeichnet, daß die Schenkelhalsschraube (19) in der Hülse (12) gegen eine Drehung um die Längsachse gesichert ist.

## Claims

1. A device (3) for the treatment of bone fractures in the proximal part of the femur in the vicinity of the joint, comprising a fixing pin (4) insertable into the bone-marrow cavity of the femur, the upper region of the fixing pin (4) having a passage opening (11) extending obliquely to its longitudinal axis, comprising a sleeve (12) secured in this passage opening in the axial direction and secured against rotation about the longitudinal axis, and comprising a femur-neck screw (19) having a shaft extending into the sleeve and axially displaceably held in the sleeve, characterised in that, on the side facing the femur-neck screw (19), the sleeve (12) protrudes so far that it extends into the femur head (2), in that, in this portion, the outer circumference of the sleeve (12) is provided with projections (23) and recesses (22) producing different diameters of the sleeve (12) in the circumferential direction, and in that the insertion depth of the femur-neck screw (19) into the sleeve (12) is limited by a stop (24) provided on the femur-neck screw (19) and positionable at a distance of between two and twelve millimetres from the sleeve (12) when the device is fitted.

2. A device according to claim 1, characterised in that the projections (23) have axially parallel sharp edges in their regions most remote from the axis.

3. A device according to claim 1 or 2, characterised in that the projections and recesses are formed by axially parallel grooves (22) arranged along the circumference of the sleeve (12).

4. A device according to claim 3, characterised in that the grooves (22) are of uniform depth over their length.

5. A device according to claim 3, characterised in that the depth of the grooves (22) decreases from the free end (21) of the sleeve (12).

6. A device according to any one of claims 3 to 5, characterised in that the grooves (22) have a curved cross-section.

7. A device according to any one of claims 3 to 6, characterised in that the width of the grooves (22) decreases from the free end (21) of the sleeve (12).

8. A device according to any one of the preceding claims, characterised in that the stop (24) is formed by the thread (20) of the femur-neck screw (19).

9. A device according to any one of the preceding claims, characterised in that the femur-neck screw (19) is freely rotatable in the sleeve (12).

10. A device according to any one of claims 1 to 8, characterised in that the femur-neck screw (19) is secured in the sleeve (12) against rotation about the longitudinal axis.

## Revendications

1. Dispositif (3) pour le traitement de fractures osseuses dans la partie proximale du fémur proche de l'articulation, comprenant une broche à verrouillage (4) qui peut être introduite dans le canal médullaire du fémur et gui présente, dans sa région supérieure, une ouverture traversante (11) s'étendant obliquement à son axe longitudinal, une douille (12) bloquée dans la direction axiale dans cette ouverture traversante et bloquée contre la rotation autour de l'axe longitudinal, et une vis de col du fémur (19) engagée dans cette douille et montée mobile en translation axiale dans la douille, caractérisé en ce que, sur le côté dirigé vers la vis de col du fémur (19), la douille (12) fait saillie suffisamment loin pour s'enfoncer dans la tête (2) du fémur, que, dans cette partie, elle porte sur sa périphérie extérieure des saillies (23) et évidements (22) qui donnent à la douille (12) des diamètres qui varient dans la direction circonférentielle, et que la profondeur d'engagement de la vis de col du fémur (19) dans la douille (12) est limitée par une butée (24) de la vis de col du fémur (19) dont la distance à la douille (12) peut être positionnée entre 2 et 12 mm au moment de la mise en place du dispositif.

2. Dispositif selon la revendication 1, caractérisé en ce que, dans leurs zones les plus éloignées de l'axe, les saillies (23) présentent des arêtes vives parallèles à l'axe.

3. Dispositif selon la revendication 1 ou 2, caractérisé en ce que les saillies et les évidements sont formés par des rainures (22) parallèles à l'axe qui sont disposées le long de la périphérie de la douille (12).

4. Dispositif selon la revendication 3, caractérisé en ce que les rainures (22) présentent une profondeur qui reste constante sur toute leur longueur.

5. Dispositif selon la revendication 3, caractérisé en ce que la profondeur des rainures (22) décroît en partant de l'extrémité libre (21) de la douille (12).

6. Dispositif selon une des revendications 3 à 5, caractérisé en ce que les rainures (22) présentent une section en forme d'arc.

7. Dispositif selon une des revendications 3 à 6, caractérisé en ce que la largeur des rainures (22) décroît en partant de l'extrémité libre (21) de la douille (12).

8. Dispositif selon une des revendications précédentes, caractérisé en ce que la butée (24) est formée par le filetage (20) de la vis de col du fémur (19).

9. Dispositif selon une des revendications précédentes, caractérisé en ce que la vis de col du fémur (19) peut tourner librement dans la douille (12).

10. Dispositif selon une des revendications 1 à 8, caractérisé en ce que la vis de col du fémur (19) est bloquée à l'encontre de la rotation autour de l'axe longitudinal à l'intérieur de la douille (12).
